(19) 

Europäisches
Patentamt
European
Patent Office
Office européen
des brevets

(11) **EP 3 888 716 A1**

(12) **EUROPEAN PATENT APPLICATION**

(43) Date of publication:
06.10.2021 Bulletin 2021/40

(51) Int Cl.:
*A61L 29/10* $^{(2006.01)}$    *A61L 31/08* $^{(2006.01)}$
*A61L 31/10* $^{(2006.01)}$    *A61L 31/16* $^{(2006.01)}$

(21) Application number: 20382268.9

(22) Date of filing: 03.04.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Tractivus SL**
**08017 Barcelona (ES)**

(72) Inventors:
• **Texidó Bartes, Robert**
  **08017 Barcelona (ES)**
• **Joan, Gilabert Porres**
  **08017 Barcelona (ES)**
• **Borrós Gómez, Salvador**
  **08017 Barcelona (ES)**

(74) Representative: **Carpmaels & Ransford LLP**
**One Southampton Row**
**London WC1B 5HA (GB)**

(54) **COATING FOR A DEVICE**

(57) The present invention relates to a coating for a device, wherein the coating comprises a polymeric film, wherein the polymeric film comprises a polymerisation product formed from a polymerisation solution comprising dopamine, or a salt thereof, and at least one amino acid, or a salt thereof; and a metallic layer formed on the polymeric film.

Figure 1

## Description

The present invention relates to a coating for a device that can have tailored properties. The invention also provides a coated device comprising said coating and methods for forming a coating on a device.

Hospital acquired infections (HAIs) represent a leading cause of death globally. All patients are susceptible to HAIs, but underlying diseases, implanted medical devices, injections, communicable diseases or recent surgeries increase the chances of HAIs. Currently about 5-10% of all hospitalizations result in a type of HAI. This affects about 5 million patients per year in the US and European Union. It is the fourth leading cause of death in the US with 210,000 patients per year. The treatment for HAIs is estimated to cost from $28-45 billion annually in the US.

It is estimated that about 70% of HAI are directly caused by the colonisation of implantable medical devices, including central line-associated bloodstream infections (11%), catheter-associated UTIs (36%), ventilator-associated pneumonia (11%) and surgical site infections (20%).

In particular, urinary catheters, venous catheter such as central venous catheter, tracheal stents, Montgomery tubes and endotracheal tube are susceptible to colonisation. All of these types of device comprise a cylindrical tube which is implanted into a patient.

The implantation of these devices can lead to a bacterial infection on the internal surface of the tube by antibiotic resistant bacteria, fungi, viruses, and other pathogens. This can cause, for instance, infections in the bloodstream, pneumonia, urinary tract infections, meningitis, or gastroenteritis.

The present invention can address these problems by providing a coating that has a surface that can have a tailored properties, one example being a tailored resistance to bacteria.

The present invention provides a coating for a device, wherein the coating comprises a polymeric film, wherein the polymeric film comprises a polymerisation product formed from a solution comprising dopamine, or a salt thereof, and at least one amino acid, or a salt thereof; and a metallic layer formed on the polymeric film.

The present invention also provides a coated device comprising a device; and a coating as described herein on at least part of a surface of the device.

It has been found that the presence of at least one amino acid, or a salt thereof, in the dopamine-containing polymerisation solution affects the properties of a metallic layer formed on the polymeric film. For example, it has been found that the presence of at least one amino acid or a salt thereof can assist in producing a stable and continuous metallic layer that is suitable for flexible substrates such as for elastomeric devices, and also affect the metallic layer's resistance to bacteria. Additionally, varying the amount of the at least one amino acid, or a salt thereof, in the polymerisation solution during the formation of the polymeric film may result in different properties of the metallic layer formed on the polymeric film providing a further ability to tailor the properties of the coating.

As stated above, the polymeric film is formed from a solution comprising dopamine, or a salt thereof, and at least one amino acid, or a salt thereof. As a result of this, the polymeric film can be polydopamine that comprises the at least one amino acid, or a salt thereof.

The geometry of the device that can be used with this invention is not particularly limited. However, it has been found that the present invention is particularly useful with devices that have interior regions, such as cylindrical shapes (for example tubes). The approach of the present invention can be used to provide tailored coatings to these hard-to-reach areas.

The device of the present invention may be a medical device. Herein, a medical device may be any device intended to be used for medical purposes. For instance, the medical device may be an implantable medical device i.e. a device that is intended to be permanently or temporarily placed within a patient's body. The implantable medical device may be a stent, a catheter, or another device in the form of a tube. In particular, the implantable medical device may be a urinary catheter, a venous catheter such as a central venous catheter, a tracheal stent, a Montgomery tube, or an endotracheal tube. In general, implantable medical devices of the invention may be implantable medical devices that comprise a substantially internal surface which is in fluid, in particular liquid, communication with the environment external to the medical device. Such internal surfaces will particularly benefit from the possible tailoring of properties provided by the present invention.

The coating is present on at least a part of a surface of the device. The coating may be present on all surfaces of the device that are in fluid communication with the environment external to the device. In this way, the benefit of the coating is achieved on all surfaces that are exposed to the external environment.

The surface upon which the coating is formed may be made of any material. For instance, the surface may be a metallic surface or a polymeric surface. Polymeric surfaces include silicones, polyurethanes and latexes. For instance, the polymeric surface may be polydimethylsiloxane.

The surface that is coated may be flexible. The coating of the present invention is particularly suited to flexible substrates. As used herein, the term flexible can refer to a material with a Young's modulus of less than 5 GPa, or less than 3 GPa, or less than 2 GPa, preferably less than 1 GPa and most preferably less than 0.5 GPa.

The polymeric film comprises a polymerisation product formed from a polymerisation solution comprising dopamine or a salt thereof and at least one amino acid or a salt thereof. The polymerisation product is the product formed from the polymerisation solution when the polymerisation solution is exposed to conditions suitable for the polymerisation of dopamine. The polymerisation solution may comprise greater than or equal to 0.001 mg/ml of the at least one amino acid or a salt thereof. The polymerisation solution may comprise greater than or equal to 0.01 mg/ml of the at least one amino acid or a salt thereof. The polymerisation solution may comprise greater than or equal to 0.1 mg/ml of the at least one amino acid or a salt thereof. The polymerisation solution may comprise greater than or equal to 1 mg/ml of the at least one amino acid or a salt thereof. Without wishing to be bound by theory, it is believed that a greater concentration of the at least one amino acid or a salt thereof in the polymerisation solution the greater its influence on the properties of the resulting polymeric film and metallic layer.

The polymerisation solution may comprise less than or equal to 50 mg/ml of the at least one amino acid or a salt thereof. The polymerisation solution may comprise less than or equal to 20 mg/ml of the at least one amino acid or a salt thereof. The polymerisation solution may comprise less than or equal to 10 mg/ml of the at least one amino acid or a salt thereof. The polymerisation solution may comprise less than or equal to 8 mg/ml of the at least one amino acid or a salt thereof. The polymerisation solution may comprise less than or equal to 6 mg/ml of the at least one amino acid or a salt thereof. The polymerisation solution may comprise less than or equal to 4 mg/ml of the at least on amino acid or a salt thereof. The polymerisation solution may comprise less than or equal to 2 mg/ml of the at least one amino acid or a salt thereof. The polymerisation solution may comprise less than or equal to 1 mg/ml of the at least one amino acid or a salt thereof. A lower concentration of the at least one amino acid or a salt thereof limits the amount of the at least one amino acid or a salt thereof to be used to a region where it has the most effective influence on the properties of the resulting coating.

The polymerisation solution may comprise greater than or equal to 0.001 mg/ml and less than or equal to 10 mg/ml of the at least one amino acid or a salt thereof. The polymerisation solution may comprise greater than or equal to 0.2 mg/ml and less than or equal to 10 mg/ml of the at least one amino acid or a salt thereof. Without wishing to be bound by theory, it was found that the presence of at least one amino acid or a salt thereof in the polymerisation solution at a concentration of greater than or equal to 0.001 mg/ml and less than or equal to 10 mg/ml during polymerisation, and particularly greater than or equal to 0.2 mg/ml affects the properties of the resulting polymeric film while limiting the total amount of the at least one amino acid that is used.

In relation to the amount of dopamine or salt thereof, and at least one amino acid or a salt thereof, the molar ratio of dopamine to the at least one amino acid is in the range of 10:1 to 1:10, preferably in the range of 5:1 to 1:5, most preferably in the range of 2:1 to 1:2.

When referring to the amounts of the at least one amino acid, this may refer to the total amount of amino acids that are present, or may refer to just one particular amino acid, for example, lysine.

The polymerisation solution may further comprise a buffer solution. The polymerisation solution may consist essentially, or consist, of dopamine or a salt thereof, at least one amino acid or a salt thereof and a buffer in a solvent. The buffer may be tris(hysroxymethyl)aminomethane (Tris), [Tris(hydroxymethyl)methylamino]propanesulfonic acid (TAPS), 2-(Bis(2-hydroxyethyl)amino)acetic acid (Bicine), N-[Tris(hydroxymethyl)methyl]glycine (Tricine), 3-[N-Tris(hydroxymethyl)methylamino]-2-hydroxypropanesulfonic acid (TAPSO), 4-(2-hydroxyethyl)-1-piperazineethanesulfonic acid (HEPES), 2-[[1,3-dihydroxy-2-(hydroxymethyl)propan-2-yl]amino]ethanesulfonic acid (TES) or 3-(N-morpholino)propanesulfonic acid (MOPS). In particular, the buffer may be Tris. The polymerisation solution may comprise a buffer at a concentration of greater than or equal to 1 mmol/L and less than or equal to 100 mmol/L.

The polymerisation solution may comprise a salt or the free base of dopamine. The dopamine salt may be dopamine hydrochloride.

The polymerisation of dopamine, or a salt thereof, may occur by any polymerisation process known in the art. For instance, the polymerisation of dopamine may be an oxidative polymerisation. Oxidative polymerisation of dopamine may be achieved by making the polymerisation solution alkaline. The oxidative polymerisation of dopamine may be achieved by adjusting the pH of the polymerisation solution to between 7 and 12, preferably between 8 and 9. When a part of the surface of a device is present in the solution during the oxidative polymerisation, the film will form on that part of the surface. The polymerisation may be allowed to proceed for between 4 and 36 hours. The polymerisation may be conducted at any suitable temperature, in particular greater than or equal to 15°C and/or less than or equal to 80°C, for example 25°C. For ease, the polymerisation may occur at room temperature. As described herein, room temperature may be 20°C.

The resulting polymeric film may have a thickness of less than or equal to 2,000 nm. The polymeric film may have a thickness of less than or equal to 1,000 nm. The polymeric film may have a thickness of less than or equal to 800 nm. The polymeric film may have a thickness of less than or equal to 500 nm. The polymeric film may have a thickness of less than or equal to 250 nm. A thinner polymeric film may be formed more quickly and require less material than a thicker polymeric film. The polymeric film may have a thickness of greater than 1 nm.

The at least one amino acid, or a salt thereof, may be at least one type of proteinogenic amino acid, or a salt thereof. Indeed, the at least one amino acid or a salt thereof of the present invention may consist of at least one proteinogenic amino acid present in *Mytildus edulis* foot protein-5 (Mefp-5), or a salt thereof, wherein Mefp-5 comprises serine, glutamic acid, lysine, glycine, proline, asparagine, alanine, histidine, and arginine.

Preferably, the at least one amino acid or a salt thereof comprises lysine or a salt thereof, or glycine or a salt thereof. The present invention may be particularly effective when the at least one amino acid, or a salt thereof, comprises lysine, or a salt thereof. The at least one amino acid, or a salt thereof, may consist of lysine or a salt thereof.

Salts of the dopamine or at least one amino acid may independently be any salts. For instance, salts of the dopamine or the at least one amino acid may be an inorganic acid salt (e.g. hydrochloride, hydrobromide, sulfate, nitrate, phosphate), a sulfonic acid salt (e.g. mesylate, esylate, isethionate, tosylate, napsylate), or a carboxylic acid salt (e.g. acetate, propionate, maleate, benzoate, salicylate, fumarate). The at least one amino acid or a salt thereof may comprise a hydrochloride salt of the at least one amino acid. For instance, the at least one amino acid or a salt thereof may consist of lysine hydrochloride.

The mussel protein Mefp-5 has remarkable adhesive properties resulting from its high 3,4-dihydroxyphenylalanine content. Without wishing to be bound to theory, the inventors of the present invention believe that the properties of the polymerisation product of dopamine, which is structurally similar to the amino acid 3,4-dihydroxyphenylalanine, or a salt thereof may also be influenced by the inclusion of at least one amino acid, or a salt thereof, from the Mefp-5 protein in the polymerisation solution.

As noted above, lysine is particularly preferred. It has been surprisingly found that the inclusion of lysine or glycine in the polymerisation solution that forms the polymeric film increases the amount of metallic coating that can be subsequently formed on the polymeric film. It has also been found that the inclusion of lysine and glycine can affect the surface morphology of the subsequent metallic coating. Accordingly, it has been found that the presence of amino acids in the polymerisation solution, such as lysine and glycine, affects the final properties of a subsequent metallic coating on the resulting polymeric film.

It has further been found that the incorporation of lysine can be used to influence the interaction of the metallic coating with bacteria. In particular, an increasing amount of lysine is associated with a greater resistance to bacteria colonisation on the metallic layer.

Without wishing to be bound to theory, it is believed that the change in bacteria resistant properties occurs due to the change in the ability of bacteria to adhere to and colonise the treated surface. The change in bacteria resistant properties can be measured relative to a reference surface. The reference surface may be an equivalent surface formed in the absence of the at least one amino acid or a salt thereof during polymerisation. Surfaces with a sufficient resistance to bacterial adhesion may be referred to as bacteriophobic.

A bacteriophobic surface of the present invention can be assessed relative to a reference surface e.g.

a surface of the present invention may have a lower number of colony forming units (CFU) than a reference surface after an *in vitro* assay of bacterial adhesion. Such a reference surface may be a metallic layer of the invention except that the polymerisation solution does not comprise at least one amino acid or a salt thereof. Alternatively, the reference surface may be polydimethylsiloxane (PDMS).

The tailored bacteria resistant properties associated with the present invention are useful for devices utilised in the medical field, referred to herein as medical devices. Medical devices utilising the present invention can help contribute to a reduction in the number of incidences of HAIs.

As noted above, the polymeric film may be formed on at least part of a device. This part of the device will then benefit from the tailored properties, such as bacteria resistant properties, conferred by the subsequent processing in line with the present invention. In this way it is possible to tailor the properties, such as bacteria resistance, to the region of the device that will benefit most from these properties. For example, in the case of a catheter, the internal surface of the tube of the catheter is particularly susceptible to bacterial colonisation and so particularly benefits from the bacteria resistant properties possible with the polymeric film and the metallic layer of the present invention. The polymeric film may be formed on at least part of the device wherein the part is a substantially internal surface which is in communication with the external environment of the device, such as the internal surface of a tube (e.g. the tubes of stents and catheters). Substantially internal surfaces of medical devices are often used to transport fluids and so are at particular risk of bacterial colonisation.

The polymeric film may be formed on at least part of a surface of the device wherein the part is an external surface of the substrate. The external surface of the device may be the outside surface of a tube, stent or catheter. The outside of a tube, stent or catheter is in direct physical contact with the tissue into which the device is implanted. Accordingly, there is a risk of HAIs resulting from bacterial colonisation on surfaces in direct physical contact with tissue. Therefore, the bacteria resistant properties that are possible with the present invention may be particularly suited to these surfaces.

The polymeric film may be formed on all surfaces of the device. This provides the ability to form tailored properties, such as bacteria-resistant properties, all over the device, which can help to contribute to a reduction in HAIs.

The metallic layer may be on all of the polymeric film or only on part of the film. Where the metallic layer is formed on part of the film, this part of the film will benefit from the tailored properties, such as bacteria resistant properties, possible with the present invention. In this way it is possible to tailor the properties to the region of the device that will benefit most from these properties. For example, in the case of a catheter, the internal surface of the tube of the catheter is particularly susceptible to bacterial colonisation and so particularly benefits from the bacteria resistant properties of the metallic layer possible with the present invention. The metallic layer may be formed on at least part of the polymeric film wherein the part is on a substantially internal surface of the substrate that is in communication with an external surface of the substrate. For instance, the substantially internal surface may be the internal surface of a tube (e.g. the internal surface of a catheter tube, the internal surface of a stent tube, the internal surface of a Montgomery tube, or the internal surface of an endotracheal tube).

The metallic layer may be formed on at least part of the polymeric film where the polymeric film is formed on an external surface of the device. The external surface of the device may be the outside surface of a tube, stent or catheter. The outside surface of a tube, stent or catheter is in direct contact with the tissue into which the device is implanted. There is a risk of HAIs resulting from bacterial colonisation of these surfaces. Therefore, the bacteria resistant properties that are possible with the present invention may be particularly suited to these surfaces.

The metallic layer of the present invention may be formed on all of the polymeric film. This provides the ability to form tailored properties, such as bacteria resistant properties, all over the area of the device covered by the polymeric film, which can help to contribute to a reduction in HAIs.

The metallic layer of the present invention may be a continuous film, i.e. there is no break in the film such that there is no region of the metallic layer that is not connected to the rest of the metallic layer. The present invention has been found to be effective at producing a continuous metallic layer rather than incorporating the metallic ions into the polymeric film. Where the metallic layer of the present invention is a continuous film, there may be no break in the bacteria resistant properties of the metallic layer. It has been found that the metallic layer wherein the metal is silver may have particularly bacteria resistant properties.

The metallic layer comprises a metal. Preferably, the metallic layer may comprise a group 11 metal (e.g. copper, silver or gold). As noted above, it is particularly preferred that the metallic layer comprises, or consists of, silver metal.

In relation to the bacteria resistance that can be demonstrated with the present invention, especially by the presence of lysine, without wishing to be bound by theory, it is believed that the ability to adjust the surface roughness and the hydrophobicity of the metallic layer allows the tailoring of these properties.

The polymeric film of the present invention provides a platform to immobilize metal via the reduction of metal ions to form a metallic layer.

The metallic layer may be present in an amount of 0.05 mg/cm$^2$ or greater. The metallic layer may be present in an amount of 0.1 mg/cm$^2$ or greater. The metallic layer may be present in an amount of 0.2 mg/cm$^2$ or greater. Herein, the amount of metallic layer refers to the mass of metallic layer per unit area of the outer surface of the metallic layer.

The coating of the invention has a surface roughness, wherein the surface roughness of the coating refers to the surface roughness of the metallic layer. The surface of the metallic layer may have a surface roughness, Ra - arithmetic average, of greater than or equal to 20 nm and/or a surface roughness, Rq - root mean squared, of greater or equal to 25 nm. The surface of the metallic layer may have a surface roughness, Ra, of greater than or equal to 50 nm. Preferably, the surface of the metallic layer has a surface roughness, Ra, of greater than or equal to 100 nm. In some embodiments, the surface of the metallic layer has a surface roughness, Rq, of greater than or equal to 50 nm. Preferably, the surface of the metallic layer has a surface roughness, Rq, of greater than or equal to 100 nm. Without wishing to be bound by theory, it is believed that a greater surface roughness may result in a greater degree of bacteria resistant properties.

Surface roughness, in the context of the present invention, can be defined by the arithmetical mean roughness (Ra) and/or the root mean squared roughness (Rq). These parameters may be interpreted in line with ISO 4287 and ISO 4288 standards.

The device may further comprise a cover layer, wherein said cover layer is formed on the metallic layer. The cover layer may be formed on a part of the metallic layer or on all of the metallic layer. The cover layer may be formed on only a part of the metallic layer. In particular, the cover layer may be formed on the metallic layer on the external surface of a stent, catheter or tube. The metallic layer on the external surface of a stent, catheter or tube may be particularly susceptible to physical damage and may particularly benefit from the physical protection provided by a cover layer. The cover layer may be removable. In this manner the cover layer can provide protection to the underlying metallic layer prior to use but then be removed to expose the metallic layer.

The cover layer may be water soluble. In this way, the cover layer may be removed when it comes into contact with a water-containing environment. The cover layer may comprise a polymer selected from polyvinyl alcohol, polyurethane, polymers from the acrylates family, or silicone polymers.

The present invention also provides a method of forming a coating on a device, wherein the method comprises: exposing at least part of a surface of a device to a polymerisation solution comprising dopamine or a salt thereof and at least one amino acid or a salt thereof, polymerising the polymerisation solution so as to form a polymeric film on the at least a part of the surface of the device; and, exposing the polymeric film to a solution comprising metal ions so as to form a metallic layer on the polymeric film.

Any features described herein in relation to the coating of the present invention, apply analogously to the method of forming the coating and vice versa.

The present invention also provides a method of forming a coating on a device, wherein the method comprises: (a) exposing at least a part of a surface of the device to a polymerisation solution comprising dopamine, or a salt thereof, and at least one amino acid, or a salt thereof; (b) polymerising the polymerisation solution so as to form a polymeric film on the at least a part of the surface of the device, wherein the pH of the polymerisation solution may be between 7 and 12;; and (c) exposing the polymeric film to a solution comprising metallic ions, such as silver ions, so as to form a metallic layer on the polymeric film.

The solution comprising metal ions may comprise any metal ions that can be reduced so as to deposit the metallic layer on the polymeric film. The metal ions in the solution may be in the + 1 oxidation state. The metal ions in the + 1 oxidation state may be group 11 metal ions in a + 1 oxidation state (e.g. Cu(I), Ag(I), or Au(I)). In a preferred embodiment, the solution comprising metal ions comprises silver ions. For instance, the solution comprising metal ions may be Tollens' reagent. Tollens' reagent is a solution comprising diamminesilver(I). The conditions that result in the reduction of the metal ions may be the presence of reducing groups. The reducing groups may consist of the reducing groups present in the polymeric film of the present invention. Where the reducing groups consist of the reducing groups present in the polymeric film, simply exposing the polymeric film to the solution comprising metal ions may result in the reduction of said metal ions and the formation of the metallic layer. The reducing groups may comprise the reducing groups present in the polymeric film and additional reducing groups added to the solution comprising metal ions. The addition of additional reducing groups may allow a quicker formation of the metallic layer.

The metallic layer may be formed on the polymeric film by exposing the polymeric film to a solution comprising metal ions, wherein the solution comprising metal ions is at greater than or equal to 40 °C and less than or equal to 120 °C. The metallic layer may be formed on the polymeric film by exposing the polymeric film to a solution comprising metal ions, wherein the solution comprising metal ions is at greater than or equal to 60 °C and less than or equal to 100 °C. For instance, the metallic layer may be formed on the polymeric film by exposing the polymeric film to a solution comprising metal ions, wherein the solution comprising metal ions is at 80 °C. The metallic layer may be formed on the polymeric film by exposing the polymeric film to a solution comprising metal ions for greater than or equal to 30 minutes and less than or equal to 8 hours. The metallic layer may be formed on the polymeric film by exposing the polymeric film to a solution comprising metal ions for greater than or equal to 1 hour and less than or equal to 4 hours. In a preferred embodiment, the metallic layer may be formed on the polymeric film by exposing the polymeric film to a solution comprising metal ions for 2 hours.

The method of the invention may comprise: exposing at least part of a surface of the device to a solution containing tris(hydroxymethyl)aminomethane (Tris buffer) at a maximum of 100 mmol/L, dopamine hydrochloride at a maximum concentration of 10 mg/ml and at least one amino acid hydrochloride salt at a maximum concentration of 100 mg/ml, and adjusting the pH of the solution to between 7 and 12; after 4-36 hours at a pH of between 7 and 12 at room temperature, washing the device with distilled water to remove any dopamine/amino acid aggregates and leaving a polymeric film (which provides a scaffold on which silver may be deposited); and forming a metallic layer on the polymeric film by heating and incubating the polymeric film in Tollens' reagent.

Tollens' reagent may be prepared starting from a silver nitrate solution, wherein the silver nitrate is present at a concentration of greater than or equal to 0.01 mol/L and less than or equal to 10 mol/L. Ammonium hydroxide (15% v/v) is added to the silver nitrate solution at a proportion of greater than or equal to 1:100 (ammonium hydroxide: silver nitrate) and less than or equal to 1:10 (ammonium hydroxide:silver nitrate). This results in the deposition of metallic silver, which forms a continuous coat. The resulting metallic layer of the present invention has a surface morphology and hydrophobicity that is tailored by the nature of the polymeric layer on which it is formed.

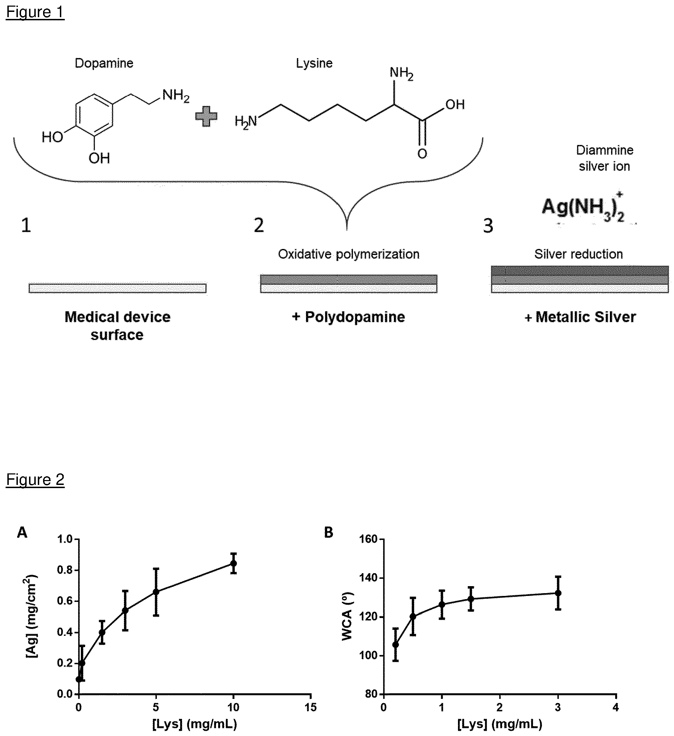

The polymeric film of the present invention may be formed by submerging at least a part of a surface of a device in a polymerisation solution comprising dopamine and lysine. The pH of the solution may then be adjusted to between 7 and 12 resulting in the oxidative polymerisation of dopamine/lysine to form a polymeric film. To form a coating of the present invention, the polymeric film may then be submerged in a Tollens' reagent. After incubation and heating while submerged in the Tollens' reagent, metallic silver has been reduced on the polymeric film to form a metallic layer. This embodiment is summarised in Figure 1.

The resulting metallic layer has surface roughness, which is believed to contribute to a superhydrophobicity. A surface may be superhydrophobic where the surface has a water contact angle of greater than or equal to 120°. It was found by the inventors that the combination of the tailored hydrophobicity and surface roughness of the metallic layer of the present invention appears to contribute to bacteria resistance.

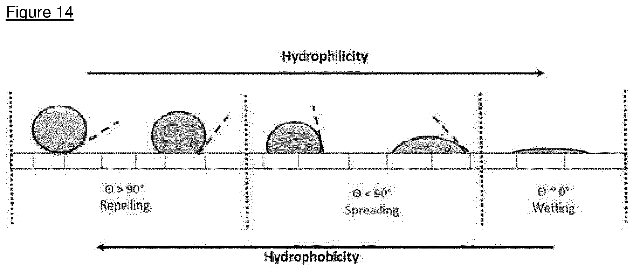

The water contact angle of a surface is determined from the angle formed between the surface and a water drop. The surface is dried under compressed air stream and a water drop is deposited on the dried surface. The water contact angle measurements are made with the sessile drop method using a Krüss DSA100 drop shape analyser. Different levels of wettability depending on contact angle are represented in Figure 14. The water contact angle is measured at 25°C.

The invention is described by the enclosed figures.

Figure 1.      An embodiment of the method of forming a coating on a device of the present invention.

Figure 2.      (A) Silver quantification normalized by surface on silicone samples as a function of lysine concentration in the polymerisation solution, and (B) Water contact angle of silver surface on silicone samples as function of lysine concentration in the polymerisation solution. Error bars indicate standard deviation (SD).

Figure 3.      FE-SEM microscopy images and confocal 3D reconstruction of surface roughness of silicone coated with metallic silver surface immobilized on polydopamine (PDA)/Lysine coatings for different lysine concentrations a) PDA only b) lysine 0.2 mg/ml c) lysine 3 mg/ml d) lysine 10 mg/ml.

Figure 4.      Roughness values (Ra and Rq) of silver coated samples with different lysine concentrations in polymerisation solution.

Figure 5.      Quantification of adhered bacteria on polydimethylsiloxane (PDMS) samples coated with different lysine concentrations. Adhesion test were performed with Gram-positive bacteria Staphylococcus aureus (MRSA) (A) and Gram-negative bacteria Pseudomonas aeruginosa (PAO1) (B). All samples were compared with PDMS uncoated as a reference of bacterial colonization. Error bars indicate SD, * indicates "significant" with $P<0.005$, ** indicates "very significant" with $P<0.001$ and n.s. indicates "not significant".

Figure 6.      End point value of optical density after 72 hours of bacterial growth curves. The bacteria (PA01 or MRSA) were exposed to different samples in order to evaluate possible antibacterial effect. The samples evaluated were PDMS as negative control and metallic layers of the invention with different lysine concentrations. Error bars indicate SD.

Figure 7.      Roughness values (Ra and Rq) of silver coated samples for different mixtures of glycine/lysine concentrations in polymerization solution.

Figure 8.      Quantification of adhered bacteria on PDMS samples coated with coatings of the invention wherein the polymeric film is formed from a polymeric solution having different glycine concentrations. Adhesion test were performed with Gram-positive bacteria Staphylococcus aureus (MRSA) (A) and Gram-negative bacteria Pseudomonas aeruginosa (PAO1) (B). All samples were compared with PDMS uncoated as a reference of bacterial colonization. Error bars indicate SD, * indicates "significant" with $P<0.005$, ** indicates "very significant" with $P<0.001$ and n.s. indicates "not significant".

Figure 9.      Bacteria adhesion quantification on a urinary Foley catheter after 15 days *in vivo*. The Foley catheters studied were a Degania© regular 2 ways Foley catheter as a control, the same catheter with an inner Bacteriophobic metallic layer (Tractivus) and a Bactiguard© Foley catheter from BARD.

Figure 10.      Bacteria quantification (CFU) of tracheal stent *in vivo* performed on a mini pig. Bacteria was quantified by bronchial washes after each period of 15 days and on the surface of the stent at the end of the study.

Figure 11.      Hemolysis test to evaluate the behavior of red blood cells in contact with the metallic layers of the invention.

Figure 12.      The water contact angle (WCA) of metallic layers of the invention comprising glycine.

Figure 13.      Confocal microscopy images of metallic silver layer on a silicone substrate without and with thickness measurements (A and B, respectively). Thickness measurements revealed a metallic layer with a thickness of near 1 $\mu$m.

Figure 14.      Representation of different levels of wettability of a droplet of water on a surface depending on the contact angle. Low wettability: Poor interaction substrate - water ($\theta > 90º$) indicating hydrophobicity, standard wettability ($\theta < 90º$) and completely wet ($\theta \sim 0º$).

**Example 1 - PDMS substrate preparation**

A polydimethylsiloxane (PDMS) substrate was prepared by mixing the two components of a Sylgard™ 184 Silicone Elastomer kit (ref 2085925) in a 10:1 (silicone elastomer:curing agent) proportion and then spreading the mixture with a paint applicator to obtain a 500 $\mu$m thick film. The film was incubated for 10 min at 150°C and, afterwards, it was cut into circles of 10 mm diameter. The substrate circles were washed and stored in aqueous solution of 70% v/v ethanol.

**Example 2** - **PDA coating**

A PDA solution was prepared by adding 0.121 g of dopamine hydrochloride (ref H852, Sigma Aldrich®) and the corresponding amount of Lysine and/or Glysine into 100 mL of 50 mM Tris buffer solution (ref Sigma Aldrich®). The pH of the solution was adjusted to basic (pH>8, preferable 10) to allow optimized self-polymerization of PDA. A PDMS film from example 1 was immersed in the dopamine solution for 6 hours at room temperature. Freshly coated membranes were rinsed with MilliQ to eliminate the excess of PDA.

**Example 3** - **silver coating**

PDA-coated samples from example 2 were immersed into a Tollens' reagent to perform the metallic coating. The Tollens' reagent was prepared by adding 1.70 g of silver nitrate (ref, Sigma Aldrich®) to 100 mL of MilliQ water. Then, a sufficient amount of 15% (v/v) aqueous ammonia solution was added under stirring to precipitate silver oxides and re-dissolve the formed silver precipitates. PDMS films were immersed on the Tollens' solution for 1.5 hours at 80°C temperature. Freshly coated membranes were rinsed with MilliQ to remove excess PDA.

**Example 4** - **Surface roughness and hydrophobicity studies**

The inventors found that the presence of at least one amino acid in the polymerisation solution during the oxidative polymerisation of dopamine increases the silver reduction process and therefore allows more silver to be deposited on the polymeric film (see Figure 2A). This higher amount of silver makes the micro-nano structure of the metallic layer more pronounced and adjusts the hydrophobic nature of the metallic layer (see Figure 2B). Both the amount of silver deposited and the hydrophobicity increase with the concentration of lysine in the polymerisation solution.

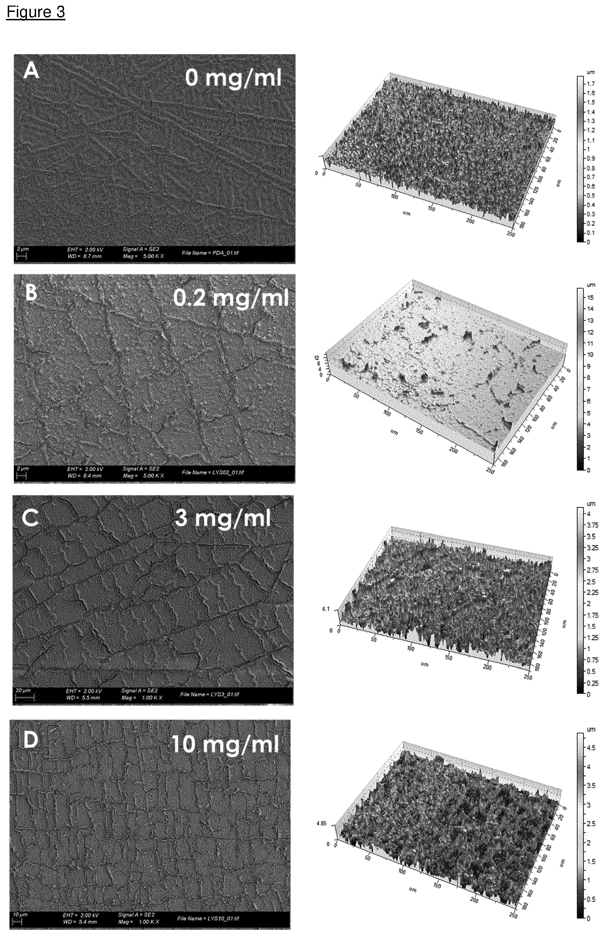

Without being confined to theory, it is believed that this increase in hydrophobicity is caused by the morphological changes to the micro-nano roughness of the surface of the metallic layer. Figure 3 shows FE-SEM microscopy images and confocal 3D reconstructions of the surface roughness of silicone coated with metallic layers of the invention with varying concentrations of lysine in the polymerisation solution.

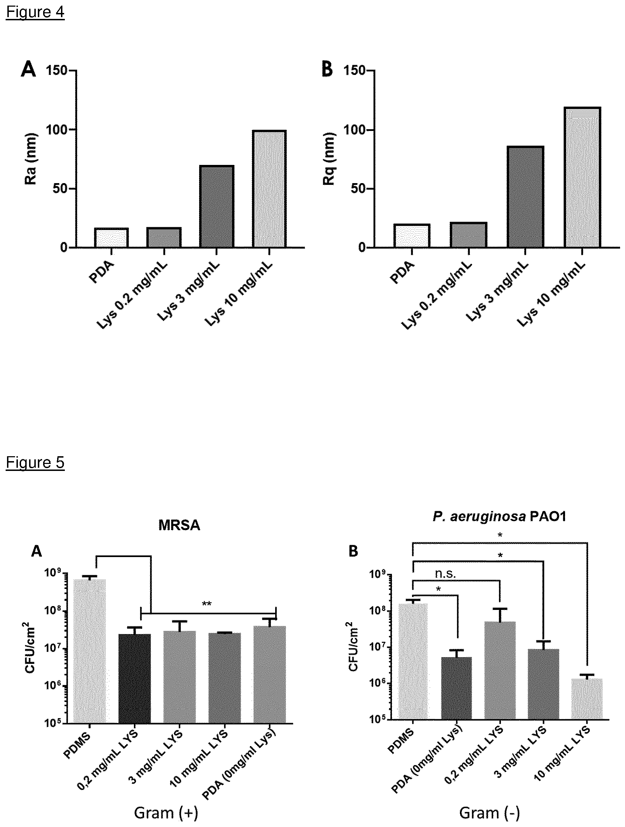

When no lysine is added to polymerization media, the sample had a flat surface with a roughness of Ra = 20 nm, which is considered a low value for roughness on the nano-scale. When the lysine concentration was increased to 0.2 mg/ml the formation of few sharp structures on the surface of the sample with a height of 5 $\mu$m was observed (Figure 3B). Higher concentration values of lysine resulted in the formation of new structures, revealing a squared-shape pattern on the silver coating (figures 3C and 3D). This pattern built has two regions of roughness: an initial nano-roughness observed for zero and near-zero amino acid concentrations and a micro-roughness observed due to the formation of the sharp structures at higher amino acid concentrations. Values of the arithmetical and quadratic mean roughness (Ra and Rq) against the concentration of lysine are presented on figures 4A and 4B respectively. The values Ra and Rq confirm the results of the FESEM and confocal images (Figure 3). i.e. increasing the amino acid concentration in the polymerisation solution increases the level of roughness of the obtained metallic layer. The presence of two orders of roughness is thought to play a critical role in increasing the hydrophobicity and bacteria resistant properties of the substrate.

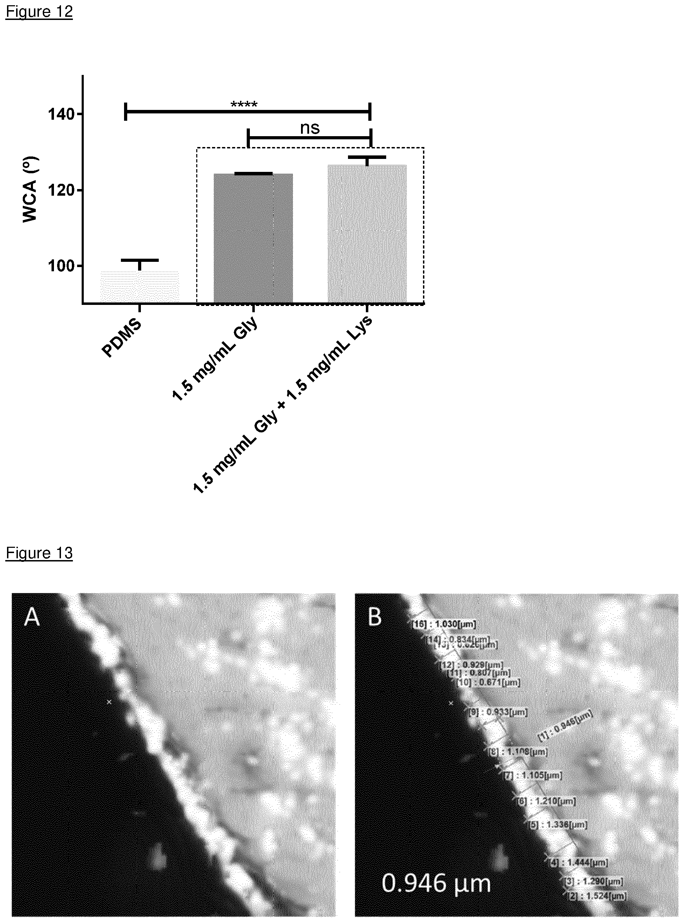

Metallic layers of the invention were also prepared from a polymerisation solution comprising glycine and from a polymerisation solution comprising glycine and lysine. The water contact angle of the surface of the resulting metallic layers is are provided in Figure 12.

**Example 5** - **FESEM and confocal microscopy**

Silver coated PDMS films of example 3 were dried under a compressed air stream and the surface morphology of the samples was studied with a field emission scanning electron microscope (Zeiss Merlin, FESEM). The surface roughness was evaluated by confocal microscopy and interferometry (Leica DCM 3D 3.3.2). Confocal images of a sample section were used to measure the metallic coating thickness. To do this, thin layers of few millimeters (preferably 3 mm) were cut from the main sample using a scalpel to obtain coupons. The coupons were placed on a sample holder support using double side adhesive tape and evaluated using confocal microscopy as shown in Figure 13. Then, an image processing software (LeicaMap 6.2) was used to obtain different measurements of the film thickness, obtaining an average near 1 $\mu$m.

From the lengths of the studied surface (mm) values, and the depth of the surface ($\mu$m) values, the arithmetic surface roughness (Ra) and the Quadratic surface roughness (Rq) could be determined. Ra is determined by the following equation:

$$Ra = \frac{1}{lb}\sum_{0}^{lb}|Z(x)|$$

wherein x is the length of the studied region, Z(x) is the depth of the studied region and $lb$ is the number of measurements performed. The results are expressed in the length units of the Z axis.

Rq is determined by the following equation:

$$Rq = \sqrt{\frac{1}{lb}\sum_{0}^{lb} Z^2(x)}$$

wherein x is the length of the studied region, $Z^2(x)$ is the square of the depth of the studied region and *lb* is the number of measurements performed. The results are expressed in the length unit of the Z-axis.

Ra and Rq values for the samples of the present invention were determined with an *lb* value of 15 and an x value of about 15 $\mu$m.

### Example 6 - Inductively coupled plasma

Inductively coupled plasma mass spectrometry analysis (ICP) was used to quantify the amount of metallic silver present in the coatings of the present invention. Silver coated PDMS films of the invention were immersed in 5 mL of MilliQ water for at least 1day. After immersion, a 1 mL sample of the water was taken and stored at 4°C until it is run on CP-OES Perkin Elmer Avio 500 to determine the amount of silver in it. From the amount of silver in the 1 mL sample, the total amount of silver in the silver coated PDMS film can be determined. ICP is a type of mass spectrometry that uses an inductively coupled plasma to ionize the sample. It atomizes the sample and creates atomic and small polyatomic ions, in this case silver ions, which are then detected

### Example 7 - Bacterial adhesion study

Metallic layers of the invention were tested in a bacterial adhesion study with both gram-positive bacteria (*Staphylococcus aureus* - MRSA) and gram-negative bacteria (*Pseudomonas aeruginosa* - PAO1). In both of these studies, silicone based samples of polydimethylsiloxane (PDMS) were used as negative controls.

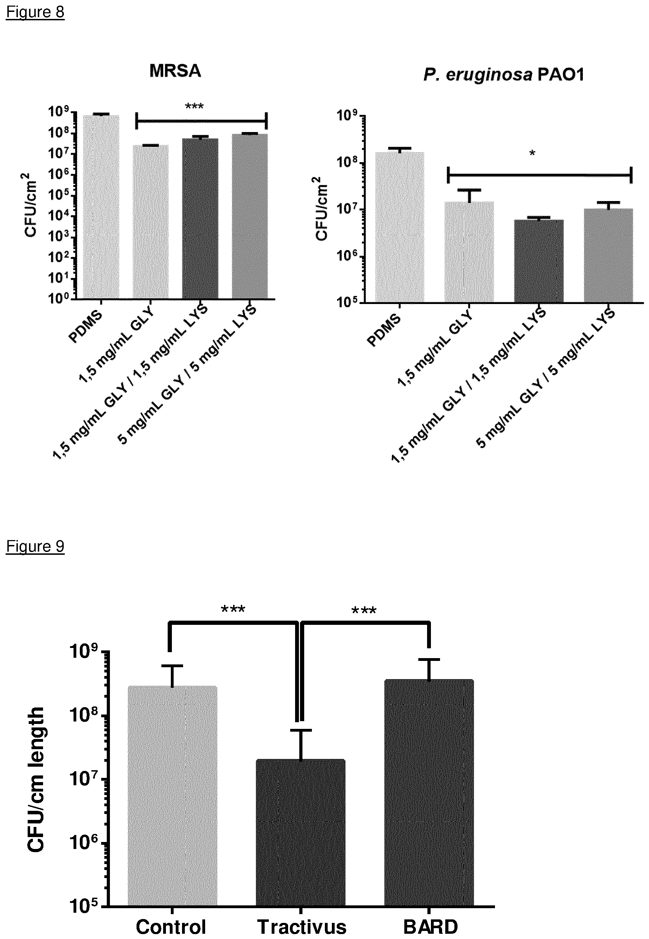

In relation to gram-positive bacteria, figures 5A and 8A demonstrate that the presence of an amino acid in the polymerisation solution results in a reduction in bacterial adhesion of up to two orders of magnitude. In relation to gram-negative bacteria, figures 5B and 8B demonstrate that the presence of an amino acid in the polymerisation solution results in a reduction in bacterial adhesion. Further, Figure 5B demonstrates that higher concentrations of amino acid in the polymerisation solution can result in a more bacteriophobic metallic layer.

### Example 8 - Antibacterial effect studies

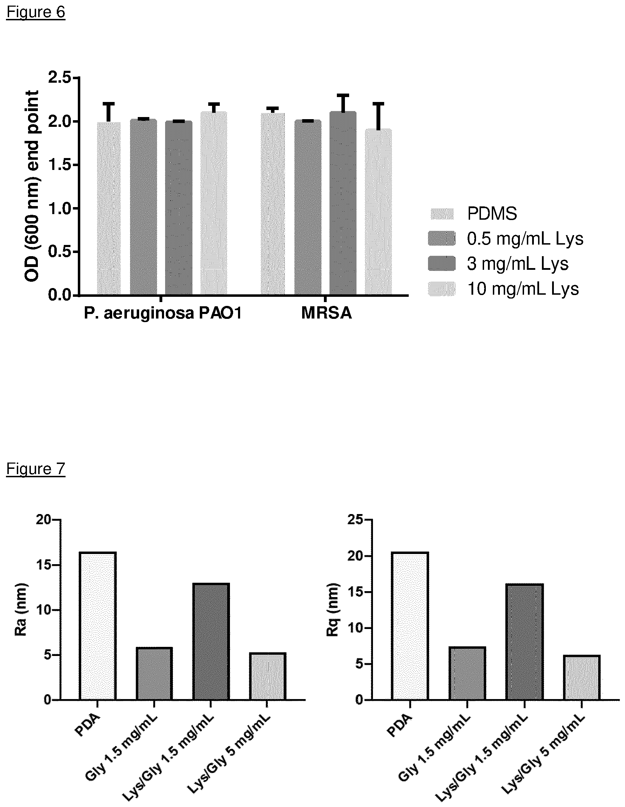

The metallic layers of the invention were tested in a bacterial growth assay to confirm that the reduction in CFU shown in the bacterial adhesion study can be attributed to a greater bacteriophobic effect. Figure 6 shows the optical density values of bacteria after 72 hours growth of a bacteria inoculum exposed to samples of metallic layers of the invention.

After 72 hours, there were no observed differences in the optical density of either gram-positive bacteria (MRSA) or gram-negative bacteria (PAO1) for any of the tested concentrations of amino acid in the polymerisation solution. Nor was there a difference in optical density between metallic layers of the invention relative to the negative control (PDMS). This confirmed that the reduction in CFU shown in the bacteria adhesion study can be attributed to a more bacteriophobic coating resisting colonisation of bacteria.

### Example 9 - Urinary catheter with bacteriophobic coating

As application proof of concept of the invention, a regular Foley urinary catheter, purchased from Degania Medical©, was coated with Bacteriophobic metallic layer in the inside. The bacteriophobic behaviour of the coated catheter was evaluated during an *in vivo* test performed in a regular pig as animal model. The coated catheter (Tractivus), the regular catheter (Control) and an Antibacterial catheter (BARD) were implanted for 15 days in groups of 6 pigs to observe the amount of bacteria attached in the device at endpoint. The results shown in Figure 9 reveals that bacterial adhesion was reduced by one order of magnitude during the test, even when compared to the antibacterial catheter.

### Example 10 - Tracheal stent with bacteriophobic coating

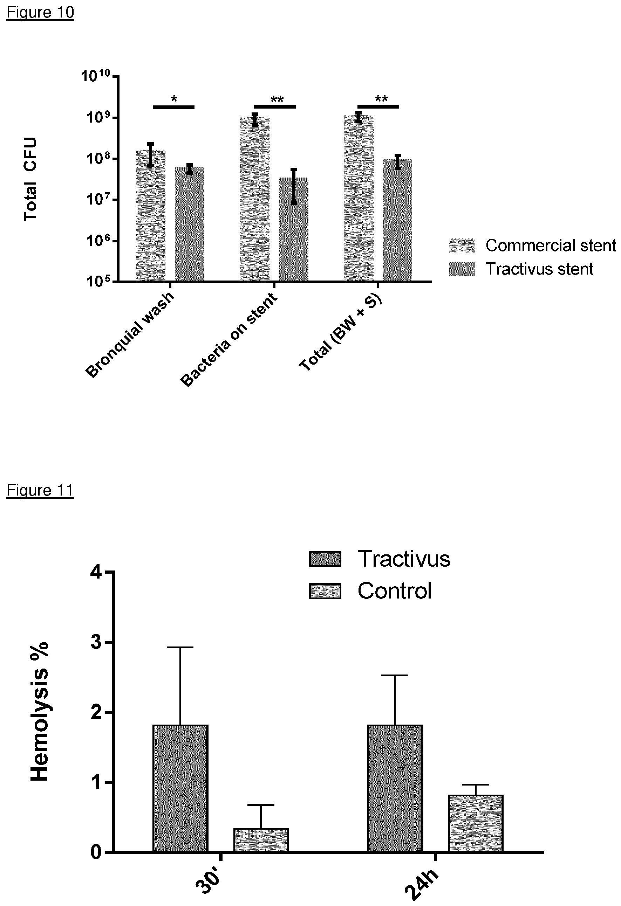

To evaluate the effectiveness of the invention, the bacteriophobic metallic layer of the invention was applied on a silicone tracheal stent in order to quantify biofilm formation during an *in vivo* test. The *in vivo* test was performed using mini pig as the animal model, where a tracheal stent was implanted to a mini pig trachea for 30 days. At day 15 and at the endpoint (30 days), a bronchial wash of the stent was performed using a flexible bronchoscope to collect the fluids from bronchial wash. Figure 10 shows the bacteria quantification of the bronchial washes and the bacteria immobilized on the surface of the explanted device after 30 days. One order of magnitude less of bacteria and no biofilm was observed on the surface of the tracheal stents with the bacteriophobic metallic layer of the invention.

### Example 11 - Central Venous Catheter

To evaluate suitability of the invention for implementation in a central venous catheter (CVC), specifically if the metallic coating presents and effect on red blood cells, haemolysis tests were carried out to confirm that the invention does not cause haemolysis when in contact with red blood cells (Figure 11). Haemolysis tests were performed by introducing blood from a healthy donor to a CVC coated with the metallic coating of the invention for a period of 30 minutes and 24 hours. The results demonstrate that the haemolysis levels present in red blood cells are below 2%. These low haemolysis values indicate that the invention can be used to avoid bacterial infection in devices that have to be implanted in the circulatory system.

The following list of embodiments forms part of the description

1. A coating for a device, wherein the coating comprises

a polymeric film, wherein the polymeric film comprises a polymerisation product formed from a polymerisation solution comprising dopamine, or a salt thereof, and at least one amino acid, or a salt thereof; and
a metallic layer formed on the polymeric film.

2. A coated device comprising

a device, and
the coating according to embodiment 1 on at least a part of a surface of the device.

3. A method of forming a coating on a device, wherein the method comprises:

a. exposing at least a part of a surface of the device to a polymerisation solution comprising dopamine, or a salt thereof, and at least one amino acid, or a salt thereof;
b. polymerising the polymerisation solution so as to form a polymeric film on the at least a part of a surface of the device; and
c. exposing the polymeric film to a solution comprising metallic ions so as to form a metallic layer on the polymeric film.

4. The coating according to embodiment 1, the coated device according to embodiment 2, or the method according to embodiment 3, wherein the at least one amino acid comprises at least one amino acid selected from the list of lysine, histidine, glycine, serine, arginine, leucine, asparagine, glutamic acid, alanine, tyrosine and proline.

5. The coating according to embodiment 1 or embodiment 4, the coated device according to embodiment 2 or embodiment 4, or the method of embodiment 3 or embodiment 4, wherein the at least one amino acid comprises at least one of lysine and glycine.

6. The coating according to any one of embodiments 1, 4 or 5, the coated device according to any one of embodiments 2, 4 or 5, or the method of any one of embodiments 3-5, wherein the at least one amino acid comprises lysine.

7. The coating according to any one of embodiments 1 or 4-6, the coated device according to any one of embodiments 2 or 4-6, or the method of any one of embodiments 3-6, wherein the coating further comprises a cover layer, wherein the cover layer is formed on the metallic layer.

8. The coating according to embodiment 7, the coated device according to embodiment 7, or the method according to embodiment 7, wherein the cover layer comprises a polymer selected from polyvinyl alcohol, polyurethane, polymers from the acrylates family, or a silicone polymer.

9. The coating according to embodiment 7 or embodiment 8, the coated device according to embodiment 7 or embodiment 8, or the method according to embodiment 7 or embodiment 8, wherein the cover layer is water soluble.

10. The coating according to any one of embodiments 1 or 4-9, the coated device according to any one of embodiments 2 or 4-9, or the method of any one of embodiments 3-9, wherein the polymeric film has a thickness of less than or equal to 1000 nm.

11. The coating according to any one of embodiments 1 or 4-10, the coated device according to any one of embodiments 2 or 4-10, or the method of any one of embodiments 3-10, wherein the metallic layer is continuous.

12. The coating according to any one of embodiments 1 or 4-11, the coated device according to any one of embodiments 2 or 4-11, or the method of any one of embodiments 3-11, wherein the metallic layer is present in an amount of 0.2 mg/cm$^2$ or greater.

13. The coating according to any one of embodiments 1 or 4-12, the coated device according to any one of embodiments 2 or 4-12, or the method of any one of embodiments 3-12, wherein the metallic layer comprises silver.

14. The coating according to any one of embodiments 1 or 4-13, the coated device according to any one of embodiments 2 or 4-13, or the method of any one of embodiments 3-13, wherein the metallic layer has a surface roughness, Ra, of greater than or equal to 20 nm and/or a surface roughness, Rq, of greater than or equal to 25 nm.

15. The coating according to any one of embodiments 1 or 4-14, the coated device according to any one of embodiments 2 or 4-14, or the method of any one of embodiments 3-14, wherein the metallic layer has a water contact angle of greater than or equal to 100°.

16. The coating according to any one of embodiments 1 or 4-15, the coated device according to any one of embodiments 2 or 4-15, or the method of any one of embodiments 3-15, wherein the metallic layer has a surface roughness, Ra, of greater than or equal to 50 nm.

17. The coating according to any one of embodiments 1 or 4-16, the coated device according to any one of embodiments 2 or 4-16, or the method of any one of embodiments 3-16, wherein the metallic layer has a surface roughness, Rq, of greater than or equal to 50 nm.

18. The coating according to any one of embodiments 1 or 4-17, the coated device according to any one of embodiments 2 or 4-17, or the method of any one of embodiments 3-17, wherein the metallic layer has a surface roughness, Ra, of greater than or equal to 100 nm.

19. The coating according to any one of embodiments 1 or 4-18, the coated device according to any one of embodiments 2 or 4-18, or the method of any one of embodiments 3-18, wherein the metallic layer has a surface roughness, Rq, of greater than or equal to 100 nm.

20. The coating according to any one of embodiments 1 or 4-19, the coated device according to any one of embodiments 2 or 4-19, or the method according to any one of embodiments 3-19, wherein the pH of the polymerisation solution is between 7 and 12.

21. The coating according to any one of embodiments 1 or 4-20, the coated device according to any one of embodiments 2 or 4-20, or the method according to any one of embodiments 3-20, wherein the concentration of the at least one amino acid or a salt thereof in the polymerisation solution is greater than or equal to 0.0001 mg/ml and less than or equal to 10 mg/ml.

22. The coating according to any one of embodiments 1 or 4-21, the coated device according to any one of embodiments 2 or 4-21, or the method according to any one of embodiments 3-21, wherein the concentration of the at least one amino acid or a salt thereof in the polymerisation solution is greater than or equal to 0.001 mg/ml.

23. The coated device according to any one of embodiments 2 or 4-22, or the method of any one of embodiments 3-22, wherein the at least part of the surface of the device is flexible.

24. The coated device according to any one of embodiments 2 or 4-23, or the method of any one of embodiments 3-23, wherein the at least part of the surface of the device is formed from a polymer.

25. The coated device according to embodiment 24, or the method according to embodiment 24, wherein the at least part of the surface of the device is formed from a silicone polymer or polyurethane.

26. The coated device of embodiment 25, or the method according to embodiment 25, wherein the at least part of the surface of the device if formed from a polydimethylsiloxane.

27. The method according to any one of embodiments 3-26, wherein the solution comprising metallic ions is Tollens' reagent.

28. A coated device obtainable by the method any one of embodiments 3-27.

**Claims**

1. A coating for a device, wherein the coating comprises
   a polymeric film, wherein the polymeric film comprises a polymerisation product formed from a polymerisation solution comprising dopamine, or a salt thereof, and at least one amino acid, or a salt thereof; and
   a metallic layer formed on the polymeric film.

2. A coated device comprising
   a device, and
   the coating according to claim 1 on at least a part of a surface of the device.

3. A method of forming a coating on a device, wherein the method comprises:

   a. exposing at least a part of a surface of the device to a polymerisation solution comprising dopamine, or a salt thereof, and at least one amino acid, or a salt thereof;
   b. polymerising the polymerisation solution so as to form a polymeric film on the at least a part of a surface of the device; and
   c. exposing the polymeric film to a solution comprising metallic ions so as to form a metallic layer on the polymeric film.

4. The coating according to claim 1, the coated device according to claim 2, or the method according to claim 3, wherein the at least one amino acid comprises at least one amino acid selected from the list of lysine, histidine, glycine, serine, arginine, leucine, asparagine, glutamic acid, alanine, tyrosine and proline.

5. The coating according to claim 1 or claim 4, the coated device according to claim 2 or claim 4, or the method of claim 3 or claim 4, wherein the at least one amino acid comprises at least one of lysine and glycine.

6. The coating according to any one of claims 1, 4 or 5, the coated device according to any one of claims 2, 4 or 5, or the method of any one of claims 3-5, wherein the at least one amino acid comprises lysine.

7. The coating according to any one of claims 1 or 4-6, the coated device according to any one of claims 2 or 4-6, or the method of any one of claims 3-6, wherein the metallic layer is continuous.

8. The coating according to any one of claims 1 or 4-7, the coated device according to any one of claims 2 or 4-7, or the method of any one of claims 3-7, wherein the metallic layer is present in an amount of 0.2 mg/cm$^2$ or greater.

9. The coating according to any one of claims 1 or 4-8, the coated device according to any one of claims 2 or 4-8, or the method of any one of claims 3-8, wherein the metallic layer comprises silver.

10. The coating according to any one of claims 1 or 4-9, the coated device according to any one of claims 2 or 4-9, or the method of any one of claims 3-9, wherein the metallic layer has a surface roughness, Ra, of greater than or equal to 20 nm and/or a surface roughness, Rq, of greater than or equal to 25 nm.

11. The coating according to any one of claims 1 or 4-10, the coated device according to any one of claims 2 or 4-10, or the method of any one of claims 3-10, wherein the metallic layer has a water contact angle of greater than or equal to 100°.

12. The coating according to any one of claims 1 or 4-11, the coated device according to any one of claims 2 or 4-11, or the method according to any one of claims 3-11, wherein the pH of the polymerisation solution is between 7 and 12.

13. The coated device according to any one of claims 2 or 4-12, or the method of any one of claims 3-12, wherein the at least part of the surface of the device is formed from a polymer.

14. The coated device according to claim 13, or the method according to claim 13, wherein the at least part of the surface of the device is formed from a silicone polymer or polyurethane.

15. A coated device obtainable by the method any one of claims 3-14.

Figure 1

Dopamine

NH₂

HO

OH

Lysine

NH₂

H₂N

OH

O

Diammine
silver ion

Ag(NH₃)₂⁺

1

2

Oxidative polymerization

3

Silver reduction

**Medical device
surface**

**+ Polydopamine**

**+ Metallic Silver**

Figure 2

A

B

Figure 3

Figure 4

Figure 5

Figure 6

Figure 7

Figure 8

Figure 9

Figure 10

Figure 11

Figure 12

Figure 13

Figure 14

Europäisches
Patentamt

European
Patent Office

Office européen
des brevets

# EUROPEAN SEARCH REPORT

**Application Number**

EP 20 38 2268

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201479<br>Thomson Scientific, London, GB;<br>AN 2014-V38325<br>XP002800446,<br>& CN 104 018 141 A (HARBIN INST TECHNOLOGY) 3 September 2014 (2014-09-03)<br>* abstract * | 1-3,7-15 | INV.<br>A61L29/10<br>A61L31/08<br>A61L31/10<br>A61L31/16 |
| A | WO 2014/204407 A1 (UNIV SINGAPORE [SG]) 24 December 2014 (2014-12-24)<br>* page 1, paragraph 1 *<br>* page 2, paragraph 4 - page 4, paragraph 5 *<br>* page 4, paragraph 8-11 *<br>* page 12, line 17 - page 13, line 36 * | 1-15 | |
| A | DATABASE WPI<br>Week 201632<br>Thomson Scientific, London, GB;<br>AN 2015-705822<br>XP002800447,<br>& CN 104 984 456 A (CHANGCHUN APPLIED CHEM INST CHINESE ACAD) 21 October 2015 (2015-10-21)<br>* abstract * | 1-15 | **TECHNICAL FIELDS SEARCHED (IPC)**<br><br>A61L |
| A | DATABASE WPI<br>Week 201969<br>Thomson Scientific, London, GB;<br>AN 2019-681740<br>XP002800448,<br>& CN 110 064 075 A (UNIV BEIJING SCI & TECHNOLOGY) 30 July 2019 (2019-07-30)<br>* abstract * | 1-15 | |

The present search report has been drawn up for all claims

| Place of search | Date of completion of the search | Examiner |
|---|---|---|
| The Hague | 23 September 2020 | Lamers, Wolfram |

EPO FORM 1503 03.82 (P04C01)

## ANNEX TO THE EUROPEAN SEARCH REPORT
## ON EUROPEAN PATENT APPLICATION NO.

EP 20 38 2268

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2020

| Patent document cited in search report | | Publication date | Patent family member(s) | | Publication date |
|---|---|---|---|---|---|
| CN 104018141 | A | 03-09-2014 | NONE | | |
| WO 2014204407 | A1 | 24-12-2014 | CN 105431181 | A | 23-03-2016 |
| | | | SG 11201510073U | A | 28-01-2016 |
| | | | WO 2014204407 | A1 | 24-12-2014 |
| CN 104984456 | A | 21-10-2015 | NONE | | |
| CN 110064075 | A | 30-07-2019 | NONE | | |

EPO FORM P0459

For more details about this annex : see Official Journal of the European Patent Office, No. 12/82